# EUROPEAN PATENT APPLICATION

(11) **EP 1 992 626 A1**
(43) Date of publication of application: **19.11.2008**
(21) Application number: 07107963.6
(22) Date of filing: 10.05.2007
(51) Int. Cl.: C07D 471/04

(54) **Process for the preparation of moxifloxacin hydrochloride**

(71) Applicant: SANDOZ AG, 4056 Basel (CH)
(72) Inventor: The designation of the inventor has not yet been filed
(74) Representative: Kröger, Bernd

(57) **Abstract**

The present invention relates to a process for the preparation of Moxifloxacin hydrochloride monohydrate, of the formula

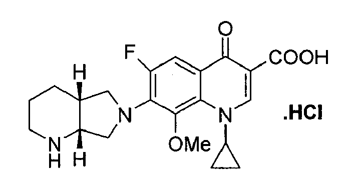

## Description

### Field of the Invention

The present invention relates to a process for the preparation of moxifloxacin hydrochloride using moxifloxacin acid addition salts. The present invention also relates to a process for the preparation of pure moxifloxacin hydrochloride monohydrate in the form of needles.

### Background of the Invention

Moxifloxacin hydrochloride, 1-cyclopropyl-7-[S,S]-2,8-diazabicyclo [4.3.0]non-8-yl)-6-fluoro,1,4-dihydro-8-methoxy-4-oxo-3-quinoline carboxylic acid hydrochloride, has the following structure: 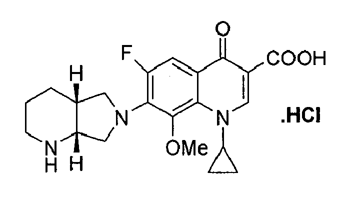 Moxifloxacin hydrochloride is a broad-spectrum antibacterial fluoroquinolone. It is particularly effective against gram-positive bacteria and is significantly better than sparfloxacin and ciprofloxacin as disclosed in European Patent 350,733 and European Patent 550,903. Moxifloxacin has activity against gram-negative and gram-positive organisms, including streptococcus pneumonia, staphylococcus aureus, pseudomonas aerugnosa, particularly against respiratory disease-causing pathogens like mycoplasma pneumonia, mycobacterium tuberculosis and chlamydia pneumonias, and the activity shown to be unaffected by β-lactamases.

U.S. Patent 5,157,117 discloses (1-cyclopropyl-6,7-difluoro-8-methoxy-4-oxo-1,4-dihydro-3-quinoline carboxylic acid-0³,0⁴)bis-(acyloxy-O) borate and a process for its preparation by reacting ethyl-1-cyclopropyl-6,7-difluoro-8-methoxy-4-oxo-1,4-dihydro-3-quinoline carboxylate with boric acid and acetic anhydride in the presence of zinc chloride, followed by its conversion to gatifloxacin hydrochloride.

Hydrates of moxifloxacin hydrochloride known are the anhydrous and monohydrate. U.S. Patent 5,849,752 discloses the monohydrate of moxifloxacin hydrochloride and its preparation by treating the anhydrous crystalline form with ethanol/ water mixtures. Further, U.S. Patent 5,849,752 discloses chromatographic purification of crude moxifloxacin followed by the preparation of anhydrous moxifloxacin hydrochloride and its further conversion to monohydrate. The process of monohydrate formation is not simple and involves heating the anhydrous moxifloxacin hydrochloride in an ethanol/water mixture at 70°C, followed by evaporation of the solvent.

European Patents 350733, 550903 and 657,448 disclose the preparation of moxifloxacin hydrochloride involving the condensation of 1-cyclopropyl-6,7-difluoro-8-methoxy-4-oxo-1,4-dihydro-3-quinoline carboxylic acid or its esters with (S,S) 2,8-diaza bicyclo[4.3.0]nonane in the presence of a base and its conversion to hydrochloride salt at higher temperatures, affording moxifloxacin and its positional isomer, (4as-cis)-1-cyclopropyl-6-(2,8-diazabicyclo [4.3.0]non-8-yl)-7-fluoro-8-methoxy-4-oxo-1,4-dihydro-3-quinoline carboxylic acid as a major impurity. As moxifloxacin and the impurity mentioned above are positional isomers, they are difficult to separate. Purification of moxifloxacin to remove this isomer results in lower yields, thereby increasing the product cost. Similarly, methods described in the prior art involves the preparation of moxifloxacin, followed by its conversion to the hydrochloride salt, thus incorporating an additional step in the manufacturing process and leading to low yields.

Accordingly, there is a need for a process that overcomes the problems encountered in the prior art process while still affording the desired product in good yield in a cost-effective manner.

### Summary of the Invention

The present invention provides a process for the preparation of pure moxifloxacin hydrochloride monohydrate comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in an organic solvent at a temperature above 50°C, in the absence of a base, to form moxifloxacin base;
b) isolating and drying the moxifloxacin base of step a);
c) treating the moxifloxacin base of step b) with an organic acid in a solvent, at a temperature of 75-80°C, to form a moxifloxacin acid addition salt;
d) treating the moxifloxacin acid addition salt of step c) with hydrochloric acid in a solvent, at a temperature of20-30°C, to form moxifloxacin hydrochloride monohydrate; and
e) isolating and drying the moxifloxacin hydrochloride monohydrate of step d) at 45-55°C, under a vacuum of 700-750 mm/Hg .

The present invention also provides a process for the preparation of a moxifloxacin acid addition salt comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in a polar, organic solvent, at a temperature above 50°C, in the absence of a base, to form moxifloxacin base;
b) isolating and drying the moxifloxacin base of step a);
c) treating the moxifloxacin base of step b) with an organic acid in a solvent, at a temperature 75-80°C, to form a moxifloxacin acid addition salt; and
d) isolating and drying the moxifloxacin acid addition salt of step c).

According to the present invention, moxifloxacin hydrochloride monohydrate is prepared in the form of needles.

The present invention provides a process for the preparation of moxifloxacin hydrochloride monohydrate that does not involve column chromatography, but rather the preparation of acid addition salts, such as L-(+)-tartarate, fumarate and di-p-toluoyl-L-tartarate, which upon acidification afford substantially pure moxifloxacin hydrochloride monohydrate.

The L-(+)-tartaric acid salt and di-p-toluoyl-L-tartarate salts are effective in depleting the impurities and depletes the chiral impurity substantially. The R,R isomer content is reduced from 5% to below 0.1 % without any further purification by using the L-(+)-tartarate salt.

A further advantage of the process of the present invention is that these acid addition salts can be easily converted to moxifloxacin hydrochloride monohydrate needles without isolation of moxifloxacin base.

The acid addition salts of moxifloxacin are directly converted in a one-pot reaction, at room temperature, to moxifloxacin hydrochloride monohydrate needles of high purity. Thus isolation of pure moxifloxacin and anhydrous moxifloxacin hydrochloride is totally avoided. As the process of the present invention does not require isolation of pure moxifloxacin, followed by its conversion to anhydrous moxifloxacin hydrochloride, the overall yield is substantially higher than the yield obtained in the prior art process.

In one aspect of the present invention, crystalline moxifloxacin L-(+)-tartarate salt is prepared.

In a further aspect of the present invention, crystalline moxifloxacin fumarate salt is prepared.

The present invention further provides a one-pot process for the preparation of moxifloxacin hydrochloride monohydrate using a moxifloxacin acid addition salt.

### Brief Description of the Drawings

Figure 1 is the X-ray powder diffractogram of crystalline moxifloxacin L-(+)-tartarate salt.
Figure 2 is the differential scanning calorimetry thermogram of crystalline moxifloxacin L-(+)-tartarate salt.
Figure 3 is the X-ray powder diffractogram of crystalline moxifloxacin fumarate salt.
Figure 4 is the differential scanning calorimetry thermogram of crystalline moxifloxacin fumarate salt.
Figure 5 shows the crystal structure of moxifloxacin hydrochloride monohydrate needles.

### Detailed Description of the Invention

The present invention provides a process for the preparation of a moxifloxacin acid addition salt comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in an organic solvent, at a temperature above 50° C, in the absence of a base, to form moxifloxacin base;
b) isolating and drying the moxifloxacin base of step a);
c) treating the moxifloxacin base of step b) with an organic acid in a solvent, at a temperature of 75-80°C, to form a moxifloxacin acid addition salt; and
d) isolating and drying the moxifloxacin acid addition salt of step c).

A suitable organic solvent for step a) includes, for example, dimethylsulphoxide, N, N'-dimethylformamide, dimethylacetamide or N-methylpyrrolidone. A suitable organic solvent for step c) includes, for example, N, N'-dimethylformamide, methanol, ethanol, isopropyl alcohol or water, or a mixture thereof.

The present invention also provides a process for the preparation of moxifloxacin hydrochloride monohydrate comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in a polar, organic solvent at a temperature above 50° C, in the absence of a base, to form moxifloxacin base;
b) isolating and drying the moxifloxacin base of step a);
c) treating the moxifloxacin base of step b) with an organic acid in a solvent, at a temperature of 75-80° C, to form moxifloxacin acid addition salt;
d) treating the moxifloxacin acid addition salt of step c) with hydrochloric acid, in a solvent, at a temperature of 20-30°C, to form moxifloxacin hydrochloride monohydrate; and
e) isolating and drying the moxifloxacin hydrochloride monohydrate of step d) at 45-55° C, under a vacuum of 700-750 mm/Hg.

A suitable organic solvent for step a) includes, for example, dimethylsulphoxide, N, N'-dimethylformamide, dimethylacetamide or N-methylpyrrolidone.

A suitable polar, organic solvent for step c) includes, for example, N, N'-dimethylformamide, methanol, ethanol, isopropyl alcohol or water, or a mixture thereof.

Hydrochloric acid as an acid source for step d) is gaseous, aqueous or dissolved in a solvent.

A suitable organic acid for step c) includes, for example, L-(+)-tartaric acid, fumaric acid or di-p-toluoyl-L-tartarate.

A suitable organic solvent for step d) includes, for example, methanol, ethanol, t-butyl alcohol, isopropyl alcohol or water, or a mixture thereof.

The present invention also provides moxifloxacin hydrochloride monohydrate is in the form of needles.

The present invention further provides a process for the preparation of moxifloxacin hydrochloride monohydrate comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in an organic solvent at a temperature above 50°C, in the absence of a base, to form moxifloxacin base;
b) adding an organic acid to the moxifloxacin base of step a);
c) stirring the reaction mass of step b) at a temperature of 75-80°C, to form moxifloxacin acid addition salt;
d) isolating and drying the moxifloxacin acid addition salt of step c);
e) treating the moxifloxacin acid addition salt of step d) with hydrochloric acid, in a solvent, at a temperature of 20-30°C, to form moxifloxacin hydrochloride monohydrate; and
f) isolating and drying the moxifloxacin hydrochloride monohydrate of step e) at 45-55° C, under a vacuum of 700-750 mm/Hg.

A suitable organic solvent for step a) includes, for example, N-N'-dimethylformamide.

A suitable organic acid for step b) includes, for example, L(+)-tartaric acid, fumaric acid or p-ditoluoyl-tartaric acid.

Hydrochloric acid as the acid source for step e) is gaseous, aqueous or dissolved in a solvent.

A suitable organic solvent for step e) includes, for example, methanol, ethanol, t-butyl alcohol, isopropyl alcohol or water, or a mixture thereof.

The present invention also provides moxifloxacin hydrochloride monohydrate in the form of needles wherein moxifloxacin base is not isolated.

Following is a reaction scheme showing the preparation of moxifloxacin acid addition salts and moxifloxacin hydrochloride monohydrate.

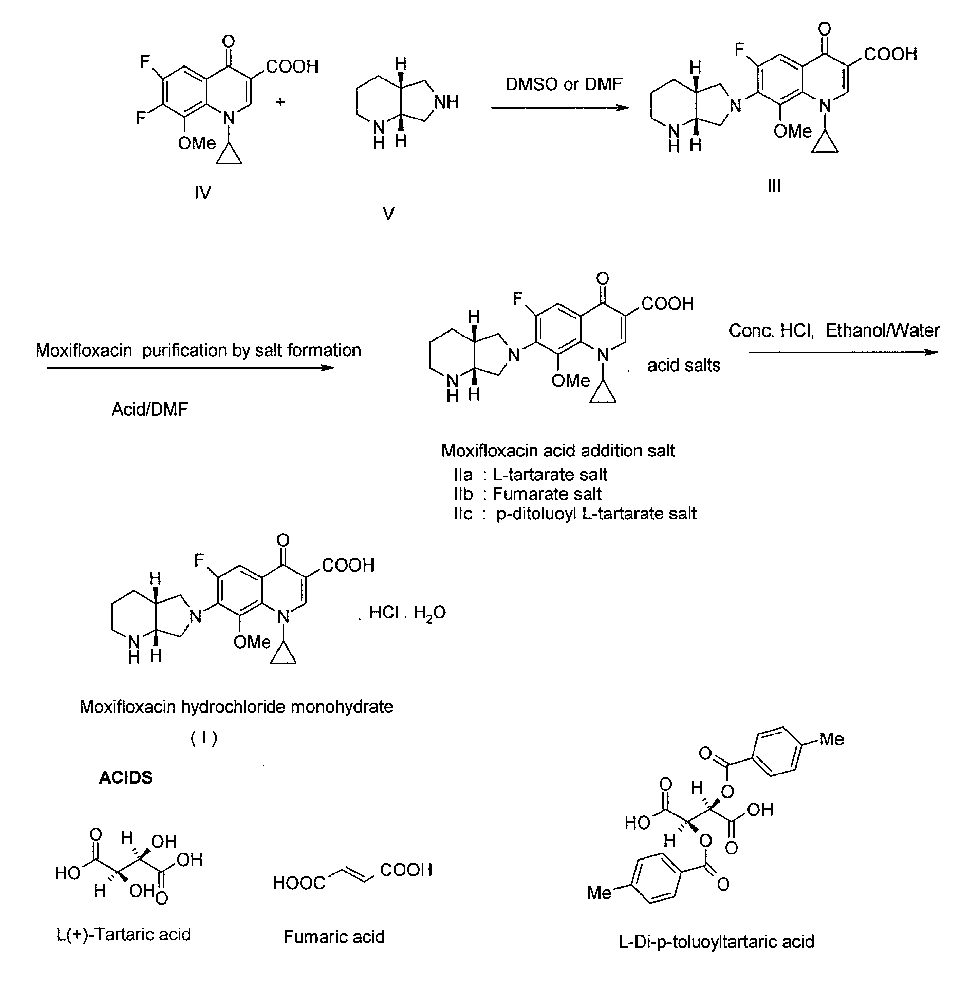

The starting materials, 1-cyclopropyl-6,7-difluoro-8-methoxy-4-oxo-1,4-dihydro-3-quinoline carboxylic acid and [S,S]-2,8-diaza-bicyclo[4.3.0]nonane, were prepared by literature reported methods.

Dimethylsulfoxide was charged to a mixture of 1-cyclopropyl-6,7-difluoro-8-methoxy-4-oxo-1,4-dihydro-3-quinoline carboxylic acid [IV] and [S,S]-2,8-diaza-bicyclo[4.3.0]nonane [V], followed by stirring at 65-70° C for 6-8 hours. Completion of the reaction is monitored by HPLC. The reaction mass was cooled to 25-30° C and water was added, followed by stirring for 2 hours. The product was filtered and the wet cake washed with water. The wet solid was dried under vacuum at 75° C till constant weight was obtained, affording 1-cyclopropyl-7-[(S,S)-2,8-diazabicyclo[4.3.0]non-8-yl]-6-fluoro-8-methoxy-4-oxo-1,4-dihydroquinoline-3-carboxylic acid (III).

N, N'-Dimethylformamide was charged to 1-cyclopropyl-7-[(S,S)-2,8-diazabicyclo[4.3.0]non-8-yl]-6-fluoro-8-methoxy-4-oxo-1,4-dihydroquinoline-3-carboxylic acid, followed by the addition of L(+)-tartaric acid at 75-80°C while stirring at 75-80° C for 3-4 hours. The reaction mass was cooled to 25-30° C and stirred for 10 12 hours. The product was filtered and the wet cake washed with N, N'-dimethylformamide. The wet solid was dried at 75°C till constant weight is obtained, affording crystalline 1-cyclopropyl-7-[(S,S)-2,8-diazabicyclo[4.3.0]non-8-yl]-6-fluoro-8-methoxy-4-oxo-1,4-dihydroquinoline-3-carboxylic acid -L-tartarate (IIa).

N, N'-Dimethylformamide was charged to 1-cyclopropyl-7-[(S,S)-2,8-diazabicyclo[4.3.0]non-8-yl]-6-fluoro-8-methoxy-4-oxo-1,4-dihydroquinoline-3-carboxylic acid, followed by the addition of fumaric acid under at 75-80° C while stirring at 75-80°C for 3-4 hours. The reaction mass was cooled to 25-30° C and stirred for 10 12 hours. The product was filtered and the wet cake washed with N, N'-dimethylformamide. The wet solid was dried at 75° C till constant weight is obtained, affording crystalline 1-cyclopropyl-7-[(S,S)-2,8-diazabicyclo[4.3.0]non-8-yl]-6-fluoro-8-methoxy-4-oxo-1,4-dihydroquinoline-3-carboxylic acid fumarate (II b).

N, N'-Dimethylformamide was charged to 1-cyclopropyl-7-[(S,S)-2,8-diazabicyclo[4.3.0]non-8-yl]-6-fluoro-8-methoxy-4-oxo-1,4-dihydroquinoline-3-carboxylic acid, followed by the addition of di-p-toluoyl-L-tartaric at 75-80° C while stirring at 75-80°C for 3-4 hours. The reaction mass was cooled to 25-30° C and stirred for 10-12 hours. The product was filtered and the wet cake washed with N, N'-dimethylformamide. The wet solid was dried at 75° C till constant weight was obtained, affording crystalline 1-cyclopropyl-7-[(S,S)-2,8-diazabicyclo[4.3.0]non-8-yl]-6-fluoro-8-methoxy-4-oxo-1,4-dihydroquinoline-3-carboxylic acid di-p-toluoyl-L-tartarate (II c).

Crystalline 1-cyclopropyl-7-[(S,S)-2,8-diazabicyclo[4.3.0]non-8-yl]-6-fluoro-8-methoxy-4-oxo-1,4-dihydroquinoline-3-carboxylic acid-L-tartarate (II a) was stirred in a mixture of ethanol and water at 25-30° C to form a clear solution, followed by the addition of concentrated HCl at 25-30° C. The solution was stirred for about 1 hour at 25-30° C. Stirring was continued at 25-30° C for 1 hour. The product was filtered and the wet cake washed with ethanol. The wet solid was dried under vacuum at 50 55° C for 3 hours, affording 1-cyclopropyl-7-[(S,S)-2,8-diazabicyclo[4.3.0]non-8-yl]-6-fluoro-8-methoxy-4-oxo-1,4-dihydroquinoline-3-carboxylic acid hydrochloride monohydrate in the form of needles.

Crystalline 1-cyclopropyl-7-[(S,S)-2,8-diazabicyclo[4.3.0]non-8-yl]-6-fluoro-8-methoxy-4-oxo-1,4-dihydroquinoline-3-carboxylic acid fumarate (IIb) was stirred in a mixture of ethanol and water at 25-30° C to form a clear solution, followed by the addition of concentrated HCl at 25-30° C. The solution was stirred for about 1 hour at 25-30° C. Stirring was continued at 25-30° C for 1 hour. The product was filtered and the wet cake washed with ethanol. The wet solid was dried under vacuum at 50 55° C for 3 hours, affording 1-cyclopropyl-7-[(S,S)-2,8-diazabicyclo[4.3.0]non-8-yl]-6-fluoro-8-methoxy-4-oxo-1,4-dihydroquinoline-3-carboxylic acid hydrochloride monohydrate in the form of needles.

Crystalline1-cyclopropyl-7-[(S,S)-2,8-diazabicyclo[4.3.0]non-8-yl]-6-fluoro-8-methoxy-4-oxo-1,4-dihydroquinoline-3-carboxylic acid di-p-toluoyl-L-tartarate (II c) is stirred in a mixture of ethanol and water at 25-30°C to form a clear solution, followed by the addition of concentrated HCl at 25-30°C. The solution was stirred for about 1 hour at 25-30° C. Stirring is continued at 25-30° C for 1 hour. The product was filtered and the wet cake washed with ethanol. The wet solid was dried under vacuum at 50 55° C for 3 hours, affording I -cyclopropyl-7-[(S,S)-2,8-diazabicyclo[4.3.0]non-8-yl]-6-fluoro-8-methoxy-4-oxo-1,4-dihydroquinoline-3-carboxylic acid hydrochloride in the form of monohydrate needles.

N, N'-Dimethylformamide was charged to a mixture of 1-cyclopropyl-6,7-difluoro-8-methoxy-4-oxo-1,4-dihydro-3-quinoline carboxylic acid [IV] and [S,S]-2,8-diaza-bicyclo[4.3.0]nonane [V], followed by stirring at 65-70°C for 6-8 hours. Completion of the reaction was monitored by HPLC. The reaction mass was cooled to 25-30° C and added L(+)-tartaric acid at 75-80° C while stirring 75-80° C for 3-4 hours. The reaction mass was cooled to 25-30° C and stirred for 10 12 hours. The product was filtered and the wet cake washed with N, N'-dimethylformamide. The wet solid was dried at 75° C till constant weight was obtained, affording crystalline 1-cyclopropyl-7-[(S,S)-2,8-diazabicyclo[4.3.0]non-8-yl]-6-fluoro-8-methoxy-4-oxo-1,4-dihydroquinoline-3-carboxylic acid-L-(+)-tartarate (II a).

The X-ray diffractogram was recorded on Phillips XPERT -PRO (MPD/MRD). The X-ray tube was equipped with a Cu-target anode, having generator settings as 45kV, 40 mA. The setting for the divergence slits was 1/4°, antiscatter slit was 1/2 °. The soller slit were 0.02 rad. The scan range (**2θ**) were from 2° - 40° with stepsize 0.01 ° time per step 16 sec.

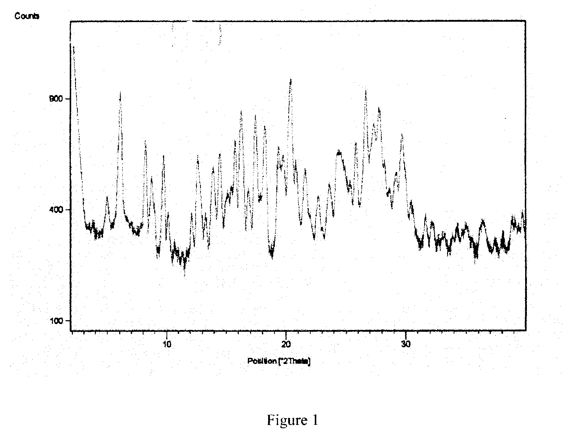

In one particular embodiment, the process of the present produces moxifloxacin L-tartarate in crystalline form. This crystalline form of moxifloxacin tartarate was characterized by an X-ray powder diffraction pattern. An example of one X-ray diffraction analysis is shown in Figure 1, and the characteristic 2-theta values (in degrees) in the X-ray diffractogram are shown in Table 1.

**Table 1 (Moxifloxacin L- (+)- tartarate)**

| **Diffraction angles (2θ,°)** | **Intensity (I/Io)** |
|---|---|
| 6.1 | 39 |
| 9.5 | 22 |
| 10.0 | 84 |
| 10.9 | 8 |
| 11.3 | 100 |
| 12.2 | 46 |
| 13.2 | 89 |
| 14.9 | 13 |
| 16.5 | 52 |
| 18.0 | 82 |
| 18.4 | 31 |
| 18.9 | 15 |
| 19.6 | 23 |
| 20.1 | 46 |
| 20.7 | 7 |
| 21.2 | 38 |
| 24.2 | 13 |
| 24.8 | 53 |
| 25.3 | 17 |
| 25.9 | 30 |
| 26.4 | 25 |
| 27.4 | 20 |
| 28.8 | 13 |

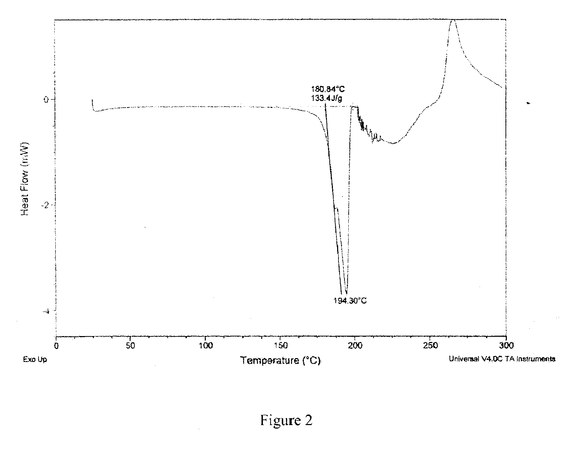

In another embodiment, the process of the present invention produces moxifloxacin fumarate in crystalline form. An example of one X-ray diffraction analysis of this crystalline form of was shown in Figure 2, and the characteristic 2-theta values (in degrees) in the X-ray diffractogram are shown in Table 2.

**Table 2 (Moxifloxacin Fumarate)**

| **Diffraction angles (2θ,°)** | **Intensity(I/Io)** |
|---|---|
| 4.2 | 4 |
| 4.9 | 14 |
| 6.1 | 84 |
| 8.2 | 55 |
| 8.7 | 24 |
| 9.7 | 45 |
| 10.1 | 13 |
| 12.1 | 16 |
| 12.6 | 42 |
| 13.3 | 9 |
| 13.9 | 31 |
| 14.5 | 41 |
| 15.0 | 13 |
| 15.4 | 15 |
| 15.7 | 45 |
| 16.2 | 71 |
| 16.8 | 18 |
| 17.4 | 70 |
| 18.2 | 63 |
| 19.3 | 44 |
| 19.7 | 35 |
| 20.3 | 100 |
| 20.8 | 28 |
| 21.5 | 30 |
| 22.7 | 12 |
| 23.5 | 18 |
| 24.3 | 23 |
| 24.7 | 25 |
| 24.9 | 6 |
| 25.8 | 40 |
| 26.6 | 77 |
| 27.3 | 42 |
| 27.8 | 52 |
| 29.7 | 42 |
| 31.6 | 8 |
| 32.2 | 8 |
| 34.9 | 7 |
| 36.4 | 11 |

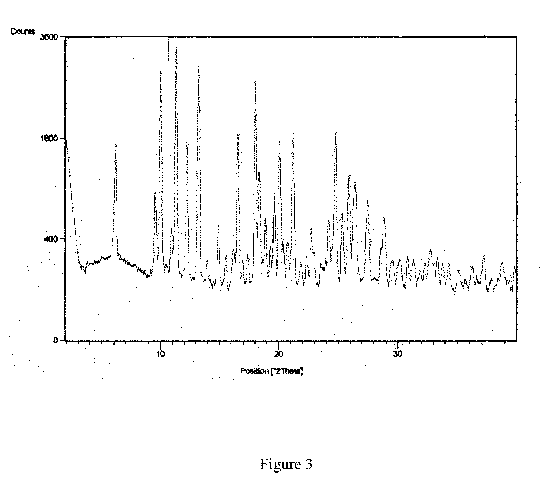

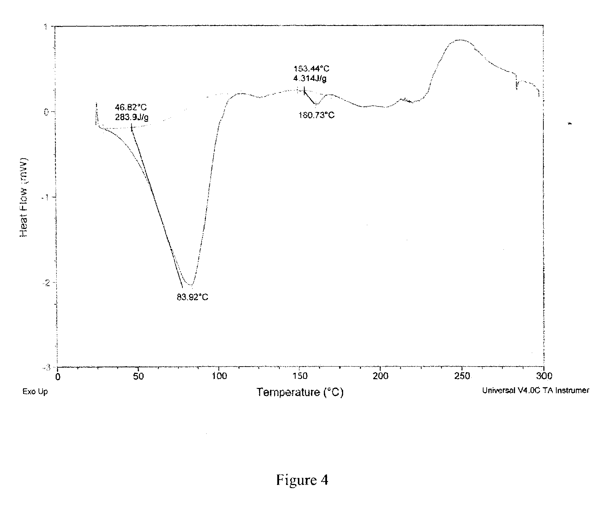

The differential scanning calorimetry (DSC) thermogram was recorded on TAQ1000 instrument with 10°/min heating rate in a nitrogen atmosphere, having empty pan as a reference with closed system. The differential scanning calorimetry (DSC) thermogram of crystalline form of moxifloxacin L-(+)-tartarate is shown in Figure 3. The differential scanning calorimetry (DSC) thermogram of the crystalline form of moxifloxacin fumarate is shown in Figure 4. It is known to one of skill in the art that the endothermic peak location may be affected by the heating rate in the DSC.

Microscopic studies were carried out using Leica DMLP with magnification (20X). The crystallides observed were needle shaped.

The FTIR spectrum was recorded on Perkin Elmer spectrum 1, KBr pellet method with resolution 4.0 cm⁻¹.

Unless defined otherwise, technical and scientific terms used herein have the same meaning as commonly understood by one of ordinary skill in the art to which this invention belongs.

The invention is further described by reference to the following examples which set forth in detail the preparation of compounds and compositions of the present invention. It will be apparent to those skilled in the art that many modifications, both to materials and methods, may be practiced without departing from the purpose and interest of this invention. The examples that follow illustrate the present invention and are not intended to limit the scope of the invention as described hereinabove or as claimed below.

### EXAMPLES

### Example 1

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid. (III)

Dimethylsulfoxide (600 ml), 50 g of 1-cyclopropyl-6,7-difluoro-8-methoxy-4-oxo-1,4-dihydro-3-quinoline carboxylic acid (IV) and 25.70 ml of [S,S]-2,8-diazabicyclo [4.3.0] nonane (V) were stirred at 65 - 70 °C for 6 - 8 hours. Completion of the reaction was monitored by HPLC. The reaction mass was cooled to 25-30 °C, 100 ml of water was added and stirred for 2 hours. The solid was filtered off with suction, washed with 100 ml water and dried under vacuum at 75°C, affording 61.0 g of the title compound.

Yield: 89.7 %
Purity: 98 % (HPLC)
Chiral impurity (R,R isomer content): 3 - 5 %

### Example 2

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid -L-(+)-tartarate (IIa)

750 ml of N, N'-Dimethylformamide, 50 g of 1-cyclopropyl-7-[(S,S)-2,8-diazabicyclo[4.3.0]non-8-yl]-6-fluoro-8-methoxy-4-oxo-1,4-dihydroquinoline-3-carboxylic acid (obtained from Example 1) and 18.7 g of L-(+)-tartaric acid were stirred at 75 - 80 °C for 3-4 hours. The reaction mass was cooled to 25 - 30°C and stirred for 10 12 hours. The solid was filtered off with suction, washed with 100 ml of N, N dimethylformamide and dried under vacuum at 75°C, affording 62.0 g of the title compound.

Yield: 90.2 %
Purity: 99.80 % (HPLC)
Chiral impurity (R,R isomer content): 0.04 %

**¹H NMR (400 MHz, DMSO)**
δ 0.80-1.30(br,m, 2H), 0.80-1.30(br,m, 2H), 1.50-1.73 (br, 2H), 1.50-1.73 (br, 2H), 2.7(m,2H), 3.02(m, 1H), 3.52(m, 2H), 3.56(s,3H), 3.9 (m, 2H), 3.97 (m,1H), 4.12 (m, 1H), 7.66(d, 1H), 8.65(s, 1H)

**¹³C NMR(400 MHz, DMSO)**
δ 21.80(CH2), 19.62(CH2), 8.22(CH₂), 9.631(CH₂), 35.25(CH), 39.51(CH), 39.92(CH), 40.65(CH), 43.04(CH₂), 55.06(CH), 56.65(CH₂), 56.70(CH₂), 61.42(CH₃), 106.49(CH), 106.2, 116.85, 136.92, 137.0, 140.25, 150.18(CH), 151.35 ,165.8, 174.43, 175.89, 175.92.

IR
3410 (N-H/O-H stretching), 2927, 2862 (aliphatic C-H stretching), 1716 (-C=O, stretching), 1623,1510( Ar C=C, stretching), 1443,1344 (aliphatic CH, bending), 1192 (C-F, stretching), 1045 (C-N, stretching), 805 (Aromatic C-H, bending)cm ⁻¹

### Example 3

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid fumarate (IIb)

500 ml of N, N-Dimethylformamide, 50 g of 1-cyclopropyl-7-[(S,S)-2,8-diazabicyclo[4.3.0]non-8-yl]-6-fluoro-8-methoxy-4-oxo-1,4-dihydroquinoline-3-carboxylic acid (obtained from Example 1) and 14.5 g of fumaric acid were stirred at 75 - 80 °C for 3-4 hours. The reaction mass was cooled to 25 - 30°C and stirred for 10-12 hours. The solid was filtered off with suction, washed with 100 ml of N, N dimethylformamide and dried under vacuum at 75°C, affording 57.50 g of the title compound.

Yield: 89.2 %
Purity: 99.75 % (HPLC)
Chiral impurity (R,R isomer content): 3 - 5 %

**¹H NMR (400 MHz, DMSO)**
δ 0.80-1.30(br,m, 2H), 0.80-1.30(br,m, 2H), 1.50-1.73 (br, 2H), 1.50-1.73 (br, 2H), 2.65-2.67(m, 2H), 3.03(m, 1H), 3.52(m, 2H), 3.56(s,3H), 3.9 (m, 2H), 3.97 (m,1H), 4.12 (m, 1H), 7.55-7.59(d,J , 1H), 8.65(s, 1H)

**¹³C NMR(400 MHz, DMSO)**
δ8.197(CH₂),9.656(CH₂),19.119(CH₂),21.540(CH₂),35.032(CH),40.606(CH),
51.935(CH₂),56.334(CH₂), 61.38(CH₃), 42.51(CH₂), 54.671(CH), 106.2(CH), 106.45(C), 116.64(C),137.05(C),136.95(C), 140.04(C),150.11(CH), 151.1(C), 165.92(C), 175.81(C).

**IR**
3428.8 (N-H/O-H stretching), 2935.58, 2862 (aliphatic C-H stretching), 1703 (-C=O, stretching), 1621.9,1576.7( Ar C=C, stretching), 1453.2,1355.9 (aliphatic CH, bending), 1189.8 (C-F, stretching), 1054.4 (C-N, stretching), 802.3 (Ar. C-H, bending) cm⁻¹

### Example 4

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid Di-p-toluoyl -L-(+)-tartarate (IIc)

150 ml of N, N-Dimethylformamide, 50 g of 1-cyclopropyl-7-[(S,S)-2,8-diazabicyclo[4.3.0]non-8-yl]-6-fluoro-8-methoxy-4-oxo-1,4-dihydroquinoline-3-carboxylic acid (obtained from Example 1) and 48.2 g of di-p-toluoyl -L-(+)- tartaric acid were stirred at 75 - 80 °C for 3-4 hours. The reaction mass was cooled to 25-30°C and stirred for 10-12 hours. The solid was filtered off with suction, washed with 100 ml of N,N-dimethylformamide and dried under vacuum at 75°C, affording 80.40 g of the title compound.

Yield: 81.93%
Purity: 99.75 % (HPLC)
Chiral impurity (R,R isomer content): 0.1 %

### Example 5

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid hydrochloride monohydrate (needles) (I) from (IIa)

50 g of 1-cyclopropyl-7-[(S,S)-2,8-diazabicyclo[4.3.0]non-8-yl]-6-fluoro-8-methoxy-4-oxo-1,4-dihydroquinoline-3-carboxylic acid -L-(+)-tartarate (IIa) (obtained from Example 2) were stirred in a mixture of 250 ml of ethanol and 250 ml of water at 25-30°C. To this solution was added 16.6 ml of concentrated HCl at 25-30°C. The resulting solution was stirred at 25-30°C for about 1 hour. The solid was filtered off with suction, washed with 100 ml of ethanol and dried under vacuum at 50 55 °C for 3 hours, affording 35.10 g of the title compound as needles.

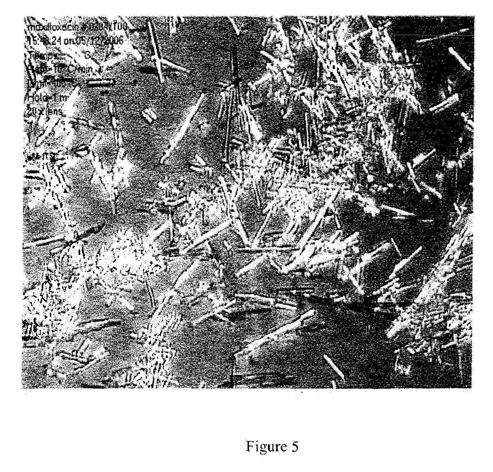

Yield: 85 %
Purity: 99.90 % (HPLC)
Water content: 3.9 %
Chiral impurity (R,R isomer content): 0.04 %
Crystal nature (needle shape): refer to Figure 5

### Example 6

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid hydrochloride monohydrate (needles) (I) from (IIb)

50 g of 1-cyclopropyl-7-[(S,S)-2,8-diazabicyclo[4.3.0]non-8-yl]-6-fluoro-8-methoxy-4-oxo-1,4-dihydroquinoline-3-carboxylic acid fumarate (II b) (obtained from Example 3) were stirred in a mixture of 250 ml of ethanol and 250 ml of water at 25-30°C. To this solution was added 16.6 ml of concentrated HCl at 25-30°C. The resulting solution was stirred at 25-30°C for about 1 hour. The solid was filtered off with suction, washed with 100 ml of ethanol and dried under vacuum at 50 55 °C for 3 hours, affording 36.0 g of the title compound as needles.

Yield: 81.82%
Purity: 99.80 % (HPLC)
Water content: 4.0 %
Chiral impurity (R,R isomer content): 3-5 %

### Example 7

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid hydrochloride monohydrate (needles) (I) from (IIc)

50 g of 1-cyclopropyl-7-[(S,S)-2,8-diazabicyclo[4.3.0]non-8-yl]-6-fluoro-8-methoxy-4-oxo-1,4-dihydroquinoline-3-carboxylic acid di- p-toluoyl -L-(+)- tartarate (II c) (obtained from Example 4) were stirred in a mixture of 250 ml of ethanol and 250 ml of water at 25-30°C. To this solution was added 16.6 ml of concentrated HCl at 25-30°C. The resulting solution was stirred at 25-30°C for about 1 hour. The solid was filtered off with suction, washed with 150 ml of ethanol and dried under vacuum at 50 55°C for 3 hours, affording 20.1g of the title compound as needles.

Yield: 69.2 %
Purity: 99.80 % (HPLC)
Water content: 3.8%
Chiral impurity (R,R isomer content): 0.1 %

### Example 8

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid -L-(+)-tartarate (IIa) (Insitu preparation without isolation of Moxifloxacin base)

750 ml of N, N-dimethylformamide, 50 g of 1-cyclopropyl-6,7-difluoro-8-methoxy-4-oxo-1,4-dihydro-3-quinoline carboxylic acid (IV) and 25.70 ml of [S,S]-2,8-diazabicyclo [4.3.0] nonane (V) were stirred at 65 - 70 °C for 6-8 hours. Completion of the reaction was monitored by HPLC. The reaction mass was cooled to 25-30 °C, 50 g of L-(+)-tartaric acid was added, and the reaction mass was stirred at 75 - 80 °C for 3-4 hours. The reaction mass was cooled to 25-30°C and stirred for 10 12 hours. The solid was filtered off with suction, washed with 150 ml of N, N-dimethylformamide and dried under vacuum at 75°C, affording 84.5 g of the title compound.
Yield: 90.32 %
Purity: 99.80 % (HPLC)
Chiral impurity (R,R isomer content): 0.06 %

### Example 9

### Preparation of 1-cyclopropyl-7-[(S,S)-2,8-diazabicyclo[4.3.0]non-8-yl]-6-fluoro-8-methoxy-4-oxo-1,4-dihydroquinoline-3-carboxylic acid hydrochloride monohydrate (needles) (I) from (IIa)

50 g of 1-cyclopropyl-7-[(S,S)-2,8-diazabicyclo[4.3.0]non-8-yl]-6-fluoro-8-methoxy-4-oxo-1,4-dihydroquinoline-3-carboxylic acid -L-(+)-tartarate (IIa) (obtained from Example 8) were stirred in a mixture of 250 ml of ethanol and 250 ml of water at 25-30°C. To this solution was added 16.6 ml of concentrated HCl at 25-30°C and continued stirring for about 1 hour at 25-30°C. The solid was filtered off with suction, washed with 100 ml of ethanol and dried under vacuum at 50 55°C for 3 hours, affording 35.10 g of the title compound as needles.

Yield: 85 %
Purity: 99.90 % (HPLC)
Water content: 3.9 %
Chiral impurity (R,R isomer content): 0.04 %
Crystal nature (needle shape): refer to Figure 5

Unless stated to the contrary, any use of words such as "including," "containing," "comprising," "having" and the like, means "including without limitation" and shall not be construed to limit any general statement that it follows to the specific or similar items or matters immediately following it. Except where the context indicates to the contrary, all exemplary values are intended for purposes of illustration. Most of the foregoing alternative embodiments are not mutually exclusive, but may be implemented in various combinations. As these and other variations and combinations of the features discussed above can be utilized without departing from the invention as defined by the claims, the foregoing description of the embodiments should be taken by way of illustration rather than by way of limitation of the invention as defined by the appended claims.

## Claims

1. A process for the preparation of moxifloxacin hydrochloride monohydrate comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in an organic solvent at a temperature above 50°C, in the absence of a base, to form moxifloxacin base;
b) isolating and drying the moxifloxacin base of step a);
c) treating the moxifloxacin base of step b) with an organic acid in a solvent, at a temperature of 75-80° C to form a moxifloxacin acid addition salt;
d) treating the moxifloxacin acid addition salt of step c) with hydrochloric acid in a solvent, at a temperature of 20-30° C, to form moxifloxacin hydrochloride monohydrate; and
e) isolating and drying the moxifloxacin hydrochloride monohydrate of step d) at 45-55° C , under a vacuum of 700-750 mm/Hg.

2. The process of claim 1 wherein said moxifloxacin hydrochloride monohydrate is in the form of needles.

3. The process of claim 1 wherein said organic solvent of step a) is dimethylsulphoxide, N, N'-dimethylformamide, dimethylacetamide or N-methylpyrrolidone.

4. The process of claim 1 wherein said solvent of step c) is N, N'-dimethylformamide, methanol, ethanol, isopropyl alcohol or water, or a mixture thereof.

5. The process of claim 1 wherein said solvent of step d) is methanol, ethanol, t-butyl alcohol, isopropyl alcohol or water, or a mixture thereof.

6. The process of claim 1 wherein said hydrochloric acid of step d) is gaseous, aqueous or dissolved in a solvent.

7. The process of claim 1 wherein said organic acid of step c) is L-(+)-tartaric acid or fumaric acid.

8. The process of claim 1 wherein said moxifloxacin acid addition salt of step c) is moxifloxacin L-(+)-tartarate or moxifloxacin fumarate.

9. The process of claim 1 wherein step e) is performed at 45-55° C.

10. The process of claim 1 wherein step e) is performed at 50°C.

11. Moxifloxacin L-(+)-tartarate in crystalline form.

12. Moxifloxacin fumarate in crystalline form.

13. Moxifloxacin L-(+)-tartarate **characterized by** a powder X-ray diffraction pattern comprising peaks at about 6.146, 9.535, 10.011,11.325, 12.230,13.224,14.88,16.534,18.028,19.621,20.060,21.181,24.757,25.904 and 27.449 (±)0.2 degrees two theta.

14. Moxifloxacin fumarate **characterized by** a powder X-ray diffraction pattern comprising peaks at about
4.975,6.068,8.185,8.730,9.704,12.656,13.913,14.466,15.715,16.235,17.411,18.178,19.35 6,19.725,20.357,20.83 5,21.5 58,24.292,25.808,26.632,27.292,27.778,29.693,(t)0.2 degrees two theta.

15. A process for the preparation of a moxifloxacin acid addition salt comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in an organic solvent, at a temperature above 50° C, in the absence of a base, to form moxifloxacin base;
b) isolating and drying the moxifloxacin base of step a);
c) treating the moxifloxacin base of step b) with an organic acid in a solvent, at a temperature of 75-80° C, to form a moxifloxacin acid addition salt; and
d) isolating and drying the moxifloxacin acid addition salt of step c).

16. The process of claim 15 wherein said organic solvent of step a) is dimethylsulphoxide, N, N'-dimethylformamide, dimethylacetamide or N-methylpyrrolidone.

17. The process of claim 15 wherein said organic solvent of step c) N, N'-dimethylformamide, methanol, ethanol, isopropyl alcohol or water, or a mixture thereof.

18. A process for the preparation of moxifloxacin hydrochloride monohydrate comprising treating a moxifloxacin acid addition salt with hydrochloric acid, in a solvent, at a temperature of 20-30°C.

19. The process of claim 18 wherein said moxifloxacin hydrochloride monohydrate is in the form of needles.

20. The process of claim 18 wherein said solvent is methanol, ethanol, t-butyl alcohol, isopropyl alcohol or water, or a mixture thereof.

21. The process of claim 18 wherein said hydrochloric acid is gaseous, aqueous or dissolved in a solvent.

22. A process for the preparation of moxifloxacin hydrochloride monohydrate comprising the steps of:
a) reacting 1-cyclopropyl-6,7-difluoro-1,4-dihydro-8-methoxy-4-oxo-3-quinoline carboxylic acid with [S,S]-2,8-diazabicyclo-[4.3.0]-nonane in an organic solvent at a temperature above 50° C, in the absence of a base, to form moxifloxacin base;
b) adding an organic acid to the moxifloxacin base of step a);
c) stirring the reaction mass of step b) with an organic acid in a solvent, at a temperature of 75-80° C, to form a moxifloxacin acid addition salt;
d) isolating and drying the moxifloxacin acid addition salt of step c);
e) treating the moxifloxacin acid addition salt of step d) with hydrochloric acid in a solvent, at a temperature of 20-30° C, to form moxifloxacin hydrochloride monohydrate; and
f) isolating and drying the moxifloxacin hydrochloride monohydrate of step e) at 45-55° C, under a vacuum of 700-750 mm/Hg.

23. The process of claim 22 wherein said moxifloxacin hydrochloride monohydrate is in the form of needles.

24. The process of claim 22 wherein said organic solvent of step a) is N, N'-dimethylformamide.

25. The process of claim 22 wherein said organic acid of step b) is L-(+)-tartaric acid or fumaric acid.

26. The process of claim 22 wherein said moxifloxacin acid addition salt of step d) is moxifloxacin L-(+)-tartarate or moxifloxacin fumarate.

27. The process of claim 22 wherein said hydrochloric acid of step e) is gaseous, aqueous or dissolved in a solvent.

28. The process of claim 22 wherein said solvent of step e) is methanol, ethanol, t-butyl alcohol, isopropyl alcohol or water, or a mixture thereof.

29. The process of claim 22 wherein step f) is performed at 45-55°C.

30. The process of claim 22 wherein step f) is performed at 50°C.

31. The process of claim 22 wherein moxifloxacin base is not isolated.
